# EUROPEAN PATENT APPLICATION

(11) **EP 1 671 595 A2**
(43) Date of publication of application: **21.06.2006**
(21) Application number: 05257636.0
(22) Date of filing: 13.12.2005
(51) Int. Cl.: A61B 17/32

(54) **Interchangeable tissue macerating and sculpting devices.**

(30) Priority: 13.12.2004 US 905044
(71) Applicant: Depuy Mitek, Inc., Norwood, MA 02062 (US)
(72) Inventor: Mcrury, Ian D., Medway, Massachusetts 02053 (US); Sengun, Mehmet Z., Framingham,Massachusetts 01702 (US); Ranucci, Kevin J., Warwick, Rhode Island 02886 (US); Dunn, Douglas W., Mansfield, Massachussets 02048 (US)
(74) Representative: Fisher, Adrian John

(57) **Abstract**

Various surgical fluid jet cutting instruments for selective bulk removal and precision sculpting of tissue are provided. In one exemplary embodiment, the instrument can include a fluid delivery tube having a nozzle for forming a high pressure fluid jet, and at least two evacuation tubes that are adapted to be selectively and removably coupled to the fluid delivery tube. Each evacuation tube can have an evacuation port or jet-receiving opening that is adapted to be positioned opposite to and spaced apart from the nozzle for receiving the high pressure fluid jet. Each evacuation tube can also be adapted for a specific use. For example, the instrument can include a first evacuation tube that is adapted to allow bulk removal of tissue, and a second evacuation tube that is adapted to allow precision sculpting of tissue.

## Description

### FIELD OF THE INVENTION

This application relates to high pressure fluid jets for macerating and sculpting tissue.

### BACKGROUND OF THE INVENTION

High pressure fluid jet systems for cutting and ablating tissue are known in the art. Fluid jet cutters focus pressurized fluid to impact desired tissue and thereby macerate the tissue. The tissue can then be suctioned or otherwise removed from the surgical site. Many devices utilize a closed-loop system that includes a collection tube positioned a distance apart from the fluid jet nozzle for collecting both the fluid jet and the removed tissue.

While known high pressure fluid jet systems are effective, they are generally limited to use in removing bulk tissue. In particular, the positioning of the fluid delivery tube relative to the collection tube on current high pressure fluid jets only allows the removal of tissue that can be positioned between the two tubes within the path of the fluid jet. The fluid collection tube prevents the user from directing the fluid jet toward tissue that is concave, flat, or even slightly convex. Precision sculpting and erosion of tissue is thus difficult to achieve.

Accordingly, there remains a need in this art for an improved high pressure fluid jet for use in bulk removal as well as precision sculpting of tissue.

### SUMMARY OF THE INVENTION

Various fluid jet cutting instruments are provided for selective bulk removal and precision sculpting of tissue. In one exemplary embodiment, a fluid jet cutting instrument is provided and it can include a fluid delivery tube having a nozzle for forming a fluid jet, and a plurality of evacuation tubes selectively and removably matable to the fluid delivery tube. Each evacuation tube can include an evacuation port that is adapted to be positioned opposite to and spaced apart from the nozzle for collecting the fluid jet from the nozzle, and the evacuation port on each evacuation tube can have a cross-sectional area measured across an opening thereof that differs from one another.

In one exemplary embodiment, the fluid jet cutting instrument can include a first evacuation tube having a first evacuation port and a second evacuation tube having a second evacuation port, and the first evacuation port can have a cross-sectional area that is greater than a cross-sectional area of the second evacuation port. In another exemplary embodiment, the evacuation port of the first evacuation tube can have a diameter that is substantially greater than a maximum diameter of a fluid jet formed by the nozzle, and the evacuation port of the second evacuation tube can have a diameter that is approximately the same as the maximum diameter of the fluid jet formed by the nozzle.

In another exemplary embodiment, a surgical fluid jet cutting instrument is provided having a fluid delivery tube with a nozzle for forming a fluid jet, and first and second evacuation tubes selectively and removably matable to the fluid delivery tube. Each evacuation tube can include a jet-receiving opening that is adapted to be positioned opposite to the nozzle for receiving a fluid jet formed by the nozzle. In one embodiment, the first evacuation tube can be adapted to allow bulk removal of tissue, and the second evacuation tube can be adapted to allow precision sculpting of tissue. In particular, in one exemplary embodiment, a cross-sectional area of the fluid-jet receiving opening of the first evacuation tube can be substantially greater than a cross-sectional area of a fluid jet formed by the nozzle, as measured at the fluid-jet receiving opening of the first evacuation tube, and a cross-sectional area of the fluid-jet receiving opening of the second evacuation tube can be approximately the same as a cross-sectional area of the fluid jet, as measured at the fluid-jet receiving opening of the second evacuation tube.

In another exemplary embodiment, a surgical fluid jet cutting instrument is provided and it can include a sheath that is slidably disposed around a fluid delivery tube and an evacuation tube. The sheath can be adapted to allow the evacuation tube to be removed and replaced.

The present invention also provides methods for selective bulk removal and precision sculpting of tissue. In one exemplary embodiment, tissue can be removed by positioning a high pressure fluid jet adjacent to a tissue surface to remove tissue in bulk. The high pressure fluid jet and tissue can be collected in a first evacuation port formed in a first evacuation tube. In an exemplary embodiment, the first evacuation port has a cross-sectional area that is substantially greater than a cross-sectional area of the high pressure fluid jet, as measured across an opening of the first evacuation port. The first evacuation tube can then be replaced with a second evacuation tube having a second evacuation port with a cross-sectional area that is approximately the same as the cross-sectional area of the high pressure fluid jet, as measured across an opening of the second evacuation port. The high pressure fluid jet can then be positioned adjacent to the tissue surface to precisely sculpt the tissue. The high pressure fluid jet and tissue can be collected in the second evacuation port in the second evacuation tube. In an exemplary embodiment, the high pressure fluid jet is formed by a nozzle on a fluid delivery tube, and the first and second evacuation tubes can be selectively and removably matable to the fluid delivery tube.

In yet another embodiment, a method for removing tissue is provided and includes coupling a first evacuation tube to a fluid delivery tube to position a first evacuation port formed in the first evacuation tube opposite to a nozzle on the fluid delivery tube. The first evacuation tube can be adapted to allow bulk removal of tissue. Tissue is then removed in bulk using a fluid jet formed by the nozzle, and the fluid jet and tissue can be collected within the first evacuation port in the first evacuation tube. The first evacuation tube is then replaced with a second evacuation tube to position a second evacuation port formed in the second evacuation tube opposite to the nozzle on the fluid delivery tube. The second evacuation tube can be adapted to allow precision sculpting of tissue. The tissue is the precisely sculpted using the fluid jet formed by the nozzle, and the fluid jet and tissue can be collected within the second evacuation port in the second evacuation tube. In one exemplary embodiment, the first evacuation port can have a cross-sectional area that is substantially greater than a cross-sectional area of the fluid jet, as measured across an opening of the first evacuation port, and the second evacuation port can have a cross-sectional area that is approximately the same as a cross-sectional area of the fluid jet, as measured across an opening of the second evacuation port. In other exemplary embodiments, the step of replacing the first evacuation tube with the second evacuation tube can include the steps of slidably removing a sheath disposed around the first evacuation tube, removing the first evacuation tube, positioning the second evacuation tube relative to the fluid delivery tube, and sliding the sheath over the second evacuation tube and the fluid delivery tube.

### BRIEF DESCRIPTION OF THE DRAWINGS

FIG. 1A is a perspective view of a distal portion of a high pressure fluid jet cutting instrument having a first evacuation tube coupled to a fluid delivery tube in accordance with one exemplary embodiment;
FIG. 1B is a perspective view of the distal portion of the high pressure fluid jet cutting instrument shown in FIG. 1A having a second evacuation tube coupled to the fluid delivery tube in accordance with another exemplary embodiment;
FIG. 2A is a perspective view of the distal portion of the high pressure fluid jet cutting instrument of FIG. 1B, showing a sheath for allowing the first and second evacuation tubes to be interchangeably coupled to the fluid delivery tube;
FIG. 2B is a cross-sectional view of the high pressure fluid jet cutting instrument shown in FIG. 2A;
FIG. 3 is a schematic illustration of a fluid jet having a cutting shear plane and a maceration zone; and
FIG. 4 is a side view of a distal portion of a high pressure fluid jet cutting instrument, showing the fluid jet positioned for precision sculpting of tissue.

### DETAILED DESCRIPTION OF THE INVENTION

Various fluid jet cutting instruments for selective bulk removal and precision sculpting of tissue are provided. In one exemplary embodiment, the instrument can include a fluid delivery tube having a nozzle for forming a high pressure fluid jet, and at least two evacuation tubes that are adapted to be selectively and removably coupled to the fluid delivery tube. Each evacuation tube can have an evacuation port or jet-receiving opening that is adapted to be positioned opposite to and spaced apart from the nozzle for receiving the high pressure fluid jet. Each evacuation tube can also be adapted for a specific use. For example, the instrument can include a first evacuation tube that is adapted to allow bulk removal of tissue, and a second evacuation tube that is adapted to allow precision sculpting of tissue. A person skilled in the art will appreciate that the instrument can include a variety of evacuation tubes adapted for specific uses, and that the exemplary features disclosed herein can be incorporated into and/or include features present in various other fluid jet cutting instruments known in the art.

The term "bulk removal" and variations thereof is intended to encompass the mass ablation of large quantities of redundant tissue such as, but not limited to fat, fat pad, plica, osteoarthritic tissue, and the term "precision sculpting" and variations thereof is intended to encompass the removal or shaping of functional anatomy which has been damaged or diseased in order to approximate the original shape and functionality. The term "macerate" and variations thereof is intended to encompass crushing between the fluid jet and a portion of the collection tube such that the tissue is ablated (almost formed into a liquefied material), and the term "cut" and variations thereof is intended to encompass removing tissue from the body using the fluid jet such that the tissue is pushed by the jet or entrained within the jet and collected in the collection tube.

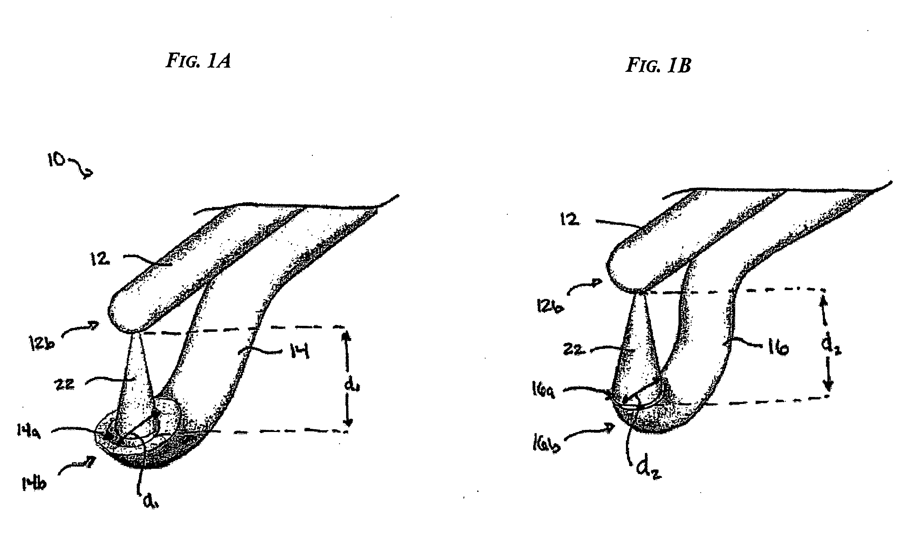

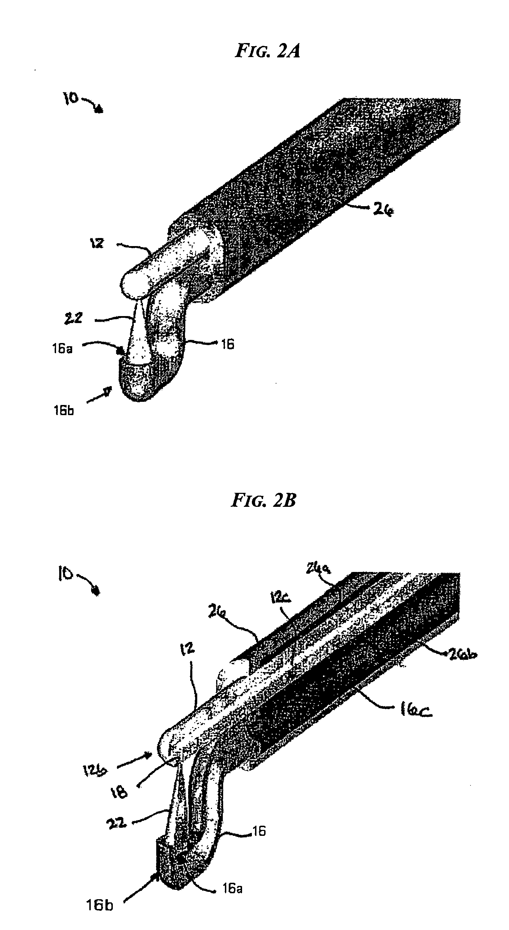

FIGS. 1A-2B illustrate one exemplary embodiment of a portion of a surgical fluid jet cutting instrument 10. As shown, the instrument 10 generally includes a fluid delivery tube 12, a first evacuation tube 14, and a second evacuation tube 16. The first and second evacuation tubes 14, 16 are selectively and removably matable to the fluid delivery tube 12 to allow an evacuation tube 14, 16 having a desired configuration to be selected based on the intended use. In particular, FIG. 1A illustrates the first evacuation tube 14 coupled to the fluid delivery tube 12. In this exemplary embodiment, the first evacuation tube 14 is adapted to allow bulk removal of tissue. In FIGS. 1B, 2A, and 2B, the first evacuation tube 14 has been removed and replaced by the second evacuation tube 16, which is shown coupled to the fluid delivery tube 12. In this exemplary embodiment, the second evacuation tube 16 is adapted to allow precision sculpting of tissue.

The fluid delivery tube 12 can have a variety of configurations, but in one exemplary embodiment it has a generally elongated shape with a proximal end (not shown), a distal end 12b, and an inner lumen 12c (shown in FIG. 2B) extending therethrough. The proximal end of the fluid delivery tube 12 can be designed to couple to a high pressure liquid source, such as a high pressure pump or liquid dispenser, for delivering fluid to the fluid delivery tube 12. The fluid delivery tube 12 can also include a nozzle 18 (shown in FIG. 2B) formed on a distal end thereof for forming and delivering a high pressure fluid jet 22. The nozzle is in communication with the inner lumen 12c such that when the proximal end of the fluid delivery tube 12 is coupled to a high pressure fluid source, fluid can be delivered through the fluid delivery tube 12 to the nozzle, which forms a fluid jet 22 having a specific shape and size.

Each evacuation tube 14, 16 can also have a variety of configurations, but in one exemplary embodiment each tube 14, 16 has a substantially elongated shape with a proximal end (not shown), a distal end 14b, 16b, and an inner lumen extending through at least a portion thereof. While not shown in FIGS. 1A-2B, the proximal end of each evacuation tube 14, 16 can be configured to couple, either directly or indirectly, to a source of suction, such as a vacuum pump, aspirator, or to a waste canister for collecting fluid and tissue evacuated through the evacuation tube 14, 16 when the tube 14, 16 is coupled to the instrument 10. The distal end 14b, 16b of each tube 14, 16 can also have a variety of configurations. However, in the illustrated exemplary embodiment, each evacuation tube 14, 16 includes an evacuation port 14a, 16a for receiving the fluid jet 22, and any tissue contained therein. The evacuation port 14a, 16a can extend into the inner lumen 14c, 16c extending through each evacuation tube 14, 16 to allow the fluid jet 22 and the tissue to be collected. The shape and size of each evacuation port 14a, 16a can also vary, as will be discussed in more detail below. In the embodiment shown in FIGS. 1A-2B, each evacuation port 14a, 16a is substantially circular in shape.

As is further shown in FIGS. 1A-1B, in an exemplary embodiment each evacuation port 14a, 16a can be adapted to be positioned a distance *d*_{*1*}*, d*_{*2*} apart from and opposite to the nozzle 18. This can be achieved, for example, by a curve formed in the distal end 14b, 16b of each evacuation tube 14, 16 such that, when each evacuation tube 14, 16 is coupled to the fluid delivery tube 12, the distal end 14b, 16b of each tube 14, 16 is spaced a distance *d*_{*1*}*, d*_{*2*} apart from the distal end 12b of the fluid delivery tube 12, as shown in FIGS. 1A-1B. While not shown, the fluid delivery tube 12 can additionally or alternatively include a curve formed therein. A person skilled in the art will appreciate that the distance *d*_{*1*}, *d*_{*2*} between the nozzle and each evacuation port 14a, 16a can vary from one another, and they can vary depending on the size of the fluid jet.

As previously indicated, each evacuation port 14, 16 can be selectively and interchangeably matable to the fluid delivery tube 12. While this can be achieved using a variety of techniques known in the art, in one exemplary embodiment the instrument 10 can include an outer housing, such as sheath 26, that is adapted to receive at least a portion of the fluid delivery tube 12 and one of the evacuation tubes 14,1 6. While the sheath 26 can have virtually any shape and size, and it can optionally be in the form of a handle to facilitate grasping of the device, in the illustrated exemplary embodiment the sheath 26 has a generally elongated shape with first and second passageways 26a, 26b extending therethrough for receiving the fluid delivery tube 12 and one of the evacuation tubes 14, 16. Each evacuation tube 14, 16 can be adapted to be removably disposed within the second passageway 26a, 26b, and a variety of techniques can be used to allow the evacuation tubes 14, 16 to temporarily mate to the sheath 26. For example, the sheath 26 can be configured to slide proximally to expose the evacuation tube, e.g., tube 16, thereby allowing the tube 16 to be removed and replaced with another evacuation tube, e.g., tube 14. The sheath 26 can then be slid distally to lock the replacement tube, e.g., tube 14, in place relative to the fluid delivery tube 12. Other exemplary mating techniques include, for example, an interference fit, a snap fit, a locking fit, a keyed fit, or any other technique that is adapted to align the evacuation tube 14, 16 with the fluid delivery tube, and that allows easy removal and replacement of the tubes 14, 16.

As was also indicated above, the first and second evacuation tubes 14, 16 can be adapted for a specific purpose, and thus each evacuation port 14a, 16a can have a variety of configurations. In one exemplary embodiment, the first evacuation tube 14 and evacuation port 14a can be configured for use in bulk removal of tissue, while the second evacuation tube 16 and evacuation port 16a can be configured for use in precision sculpting of tissue. More particularly, the first evacuation tube 14 can have an evacuation port 14a with a size, e.g., a cross-sectional area or an extent, such as a diameter d₁, measured across an opening thereof, that is larger than a size, e.g., a cross-sectional area or extent, such as a diameter d₂, measured across an opening of the second evacuation port 16a. The first evacuation port 14a can also have a size that is larger than a size of the fluid jet 22 formed by the nozzle 18, as measured when received across the opening of the evacuation port 14a, and the second evacuation port 16a can have a size that is substantially the same as, , or only slightly larger than, the size of the fluid jet 22 formed by the nozzle 18, as measured when received across the opening of the evacuation port 16a. The relatively large size of the first evacuation port 14a may prevent precision sculpting of tissue, but it will allow bulk removal of tissue, as will be discussed in more detail below. Conversely, the relatively small size of the second evacuation port 16a will allow precision sculpting of tissue, as will be discussed in more detail below.

While the size of each evacuation port 14a, 16a can vary, in one exemplary embodiment each evacuation port 14a, 16 can be configured such that the fluid jet 22 occupies a predetermined area of the evacuation port 14a, 16a. While this predetermined area can vary depending on the intended use, in one exemplary embodiment the fluid jet 22 can occupy only a portion, e.g., less than 80%, and more preferably about 50% to 60%, of the evacuation port 14a on the first evacuation tube 14, while it can occupy substantially all, e.g., more than about 90% of the evacuation port 16a of the second evacuation tube 16. Such a configuration allows the second evacuation tube 16 to have a relative small size, thus allowing the fluid jet 22 to be positioned tangential to the tissue surface without interference from the tube 16, as will be discussed in more detail below.

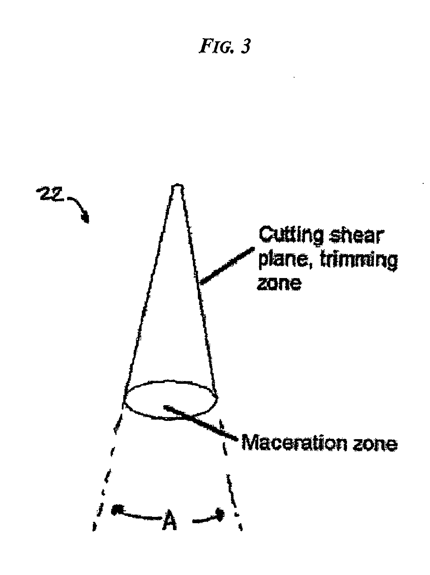

As noted above, the desired area of the each evacuation port 14a, 16a to be occupied by the fluid jet 22 can vary depending on the size of the fluid jet 22 and the distance *d*_{*1*}*, d*_{*2*} between the nozzle 18 and each evacuation port 14a, 16a. In one exemplary embodiment, the fluid jet 22 can be configured to have a cone angle *A*, shown in FIG. 3, that is in the range of about 15° to 20°, and more preferably that is about 17° to 19°, and the distance *d*_{*1*}, *d*_{*2*} can be in the range of about 1 mm to 5 mm. The distance *d*_{*1*}, *d*_{*2*} between the nozzle 18 and each evacuation port 14a, 16a can be the same, or it can optionally vary. The pressure of the fluid jet 22 can also vary, but in an exemplary embodiment the fluid jet 22 is delivered at a pressure that is in the range of about 1000 PSI to 20,000 PSI, more preferably 5000 PSI to 15,000 PSI.

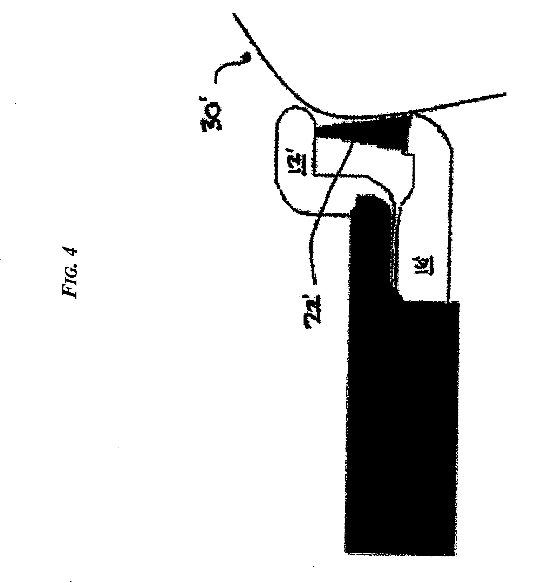

In use, the first evacuation tube 14 can be used for bulk removal of tissue, and the second evacuation tube 16 can be used for precision sculpting of tissue. First, referring to FIG. 3, fluid 22 jet is shown in more detail, and as shown the fluid jet 22 includes a shear cutting plane which is formed around a perimeter thereof along a length thereof, and a maceration zone, which is internal to the cutting plane. The first evacuation tube 14 allows the fluid jet 22 to be positioned such that the shear cutting plane is transverse to the tissue surface, i.e., it extends into the tissue surface, thus allowing the fluid jet 22 to be used for bulk removal of tissue such that the tissue within the maceration zone will be macerated. The first evacuation tube 14 can then be removed and replaced with the second evacuation tube 16, which allows the fluid jet 22 to be positioned such that the shear cutting plane is substantially tangential to the tissue surface, thus allowing the fluid jet 22 to be used for precision sculpting of tissue. FIG. 4 illustrates one embodiment of a fluid jet cutting instrument 10' having a fluid delivery tube 12' and an evacuation tube 16', that are similar to fluid delivery tube 12 and evacuation tube 16, showing a fluid jet 22' positioned such that the shear cutting plane is substantially tangential to the tissue surface, thus allow the fluid jet 22' to be used for precision sculpting of tissue. Accordingly, by providing selectively interchangeable evacuation tubes 14, 16, the fluid jet 22 can be selectively positioned for use in bulk removal of tissue and for use in precision sculpting of tissue.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A fluid jet cutting instrument, comprising:
a fluid delivery tube having a nozzle for forming a fluid jet;
a plurality of evacuation tubes selectively removably matable to the fluid delivery tube, each evacuation tube having an evacuation port adapted to be positioned opposite to and spaced apart from the nozzle for collecting a fluid jet formed by the nozzle, and the evacuation port on each evacuation tube having a cross-sectional area measured across an opening thereof that differs from one another.

2. The instrument of claim 1, wherein the plurality of evacuation tubes comprise a first evacuation tube having a first evacuation port, and a second evacuation tube having a second evacuation port, the cross-sectional area of the first evacuation port being greater than the cross-sectional area of the second evacuation port.

3. The instrument of claim 2, wherein the first evacuation port has a diameter measured across the opening thereof that is substantially greater than a maximum diameter of a fluid jet formed by the nozzle, and the second evacuation port has a diameter measured across the opening thereof that is approximately the same as the maximum diameter of the fluid jet formed by the nozzle.

4. The instrument of claim 2, wherein the first evacuation tube is adapted to allow bulk removal of tissue, and wherein the second evacuation tube is adapted to allow precision sculpting of tissue.

5. The instrument of claim 1, further comprising a sheath adapted to be slidably disposed around the fluid delivery tube and one of the plurality of evacuation tubes for allowing the evacuation tube to be selectively replaced.

6. The instrument of claim 1, wherein the evacuation port in each of the plurality of evacuation tubes comprises a substantially circular opening extending into a lumen formed through the evacuation tube.

7. A surgical fluid jet cutting instrument, comprising:
a fluid delivery tube having a nozzle for forming a fluid jet; and
first and second evacuation tubes selectively and removably matable to the fluid delivery tube, each evacuation tube including a jet-receiving opening adapted to be positioned opposite to the nozzle for receiving a fluid jet formed by the nozzle;
wherein the first evacuation tube is adapted to allow bulk removal of tissue, and wherein the second evacuation tube is adapted to allow precision sculpting of tissue.

8. The instrument of claim 7, wherein a fluid jet formed by the nozzle has a cross-sectional area, as measured at the fluid-jet receiving opening, that is substantially less than a cross-sectional area of the fluid-jet receiving opening of the first evacuation tube, and that is approximately the same as a cross-sectional area of the fluid-jet receiving opening of the second evacuation tube.

9. The instrument of claim 8, wherein the first evacuation tube is adapted to receive a fluid jet formed by the nozzle at a substantial mid-portion thereof.

10. The instrument of claim 7, further comprising a sheath disposed around the fluid delivery tube and adapted to selectively and removably receive one of the first and second evacuation tubes.

11. The instrument of claim 10, wherein the sheath is slidable relative to the fluid delivery tube.

12. The instrument of claim 7, wherein the evacuation port in the first and second evacuation tubes comprises a substantially circular opening extending into a lumen formed through the evacuation tube.
